(19) Europäisches Patentamt
European Patent Office
Office européen des brevets

(11) Veröffentlichungsnummer : **0 129 774**
**B1**

(12) # EUROPÄISCHE PATENTSCHRIFT

(45) Veröffentlichungstag der Patentschrift : **10.09.86**

(51) Int. Cl.⁴ : **C 07 C 45/75**, C 07 C 47/198, C 07 H 3/08

(21) Anmeldenummer : **84106727.5**

(22) Anmeldetag : **13.06.84**

(54) **Verfahren zur Herstellung von Aldolen.**

(30) Priorität : **15.06.83 DE 3321517**

(43) Veröffentlichungstag der Anmeldung : **02.01.85 Patentblatt 85/01**

(45) Bekanntmachung des Hinweises auf die Patenterteilung : **10.09.86 Patentblatt 86/37**

(84) Benannte Vertragsstaaten :
**BE CH DE FR GB LI NL**

(56) Entgegenhaltungen :
**FR-A- 1 044 694**
**FR-A- 2 385 670**
**CHEMICAL ABSTRACTS, RN : 85030-53-1, Columbus, Ohio, US;**
**BULLETIN DE LA SOCIETE CHIMIQUE DE FRANCE, 1972, Nr. 11, pages 4308-4314, Nr. 677; J. JUSTIN et al.: "Transformation thermique de dioxannes-1,3 substitués en 2 et 5"**
**ACTA CHEMICA SCANDINAVICA, Band 25, 1971, Seiten 2211-2216; H. BERTILSKÖLD et al.: "The formaldehyde species reacting in base-catalyzed aldol condensations"**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Merger, Franz, Dr.**
**Max-Slevogt-Strasse 25**
**D-6710 Frankenthal (DE)**
Erfinder : **Hettinger, Peter, Dr.**
**Schloss-Strasse 3**
**D-6803 Edingen-Neckarhausen (DE)**
Erfinder : **Lange, Arno, Dr.**
**Oberes Geistal 3 b**
**D-6702 Bad Duerkheim (DE)**

**EP 0 129 774 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Für die Herstellung substituierter Ethermalonsäuren der allgemeinen Formel (II)

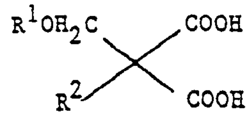

(II)

in der $R^1$ einen verzweigten oder unverzweigten Alkylrest mit bis zu 5 C-Atomen darstellt und $R^2$ einen verzweigten oder unverzweigten Alkylrest mit bis zu 6 C-Atomen bedeutet — es handelt sich um Vorprodukte für wichtige Pflanzenschutzmittel — werden entsprechende Aldole der allgemeinen Formel (I)

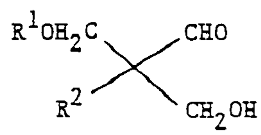

(I)

benötigt. Diese sollten sich nach einem für ähnliche Stoffe bekannten Verfahren, Oxidation mit Salpetersäure schon bei einer Temperatur von 20 bis 40 °C in Gegenwart eines Metallsalzes der VII. Nebengruppe in die gesuchten Ethermalonsäuren überführen lassen (vgl. DE-OS 15 68 227).

Ethermalonsäuren konnten bisher nur auf einem Weg erhalten werden, der weder wirtschaftlich noch sicherheitstechnisch brauchbar scheint. Nach Ar. 297 (4), 219 (1964) lassen sich monoalkylsubstituierte Malonester, die bereits selbst schwierig darstellbar und somit wirtschaftlich wenig attraktiv sind, mit den bekannt toxischen und problematisch zu handhabenden Chlormethyl-alkylethern zu disubstituierten Alkoximethyl-alkylmalonestern umsetzen.

Die Suche nach einer einfachen technischen Synthese substituierter Ethermalonsäuren war daher aus wirtschaftlichen und sicherheitstechnischen Aspekten geboten. Zum anderen waren die Ausgangsstoffe für ein Verfahren nach dem Vorschlag der DE-OS 15 68 227, nämlich Alkoximethyl-alkylhydroxipropanale praktisch nicht verfügbar.

Ähnliche Verbindungen dieses Typs werden zwar als Intermediärstufen der Bildung von Etheralkoholen aus Alkanalen und Formaldehyd in Gegenwart von Alkoholen mit Natronlauge angenommen, nämlich

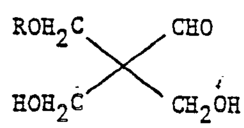

jedoch sind auch diese Verbindungen nicht explizit beschrieben (Acta Chem. Scand. *25*, 2211 (1971)).

Die entsprechenden Etherdiole, die ebenfalls als Ausgangsstoffe zur Herstellung der substituierten Ethermalonsäuren in Betracht kommen, sind ihrerseits praktisch nicht zugänglich, da sie nur entweder durch Reduktion der (wie erwähnt, schlecht zugänglichen) Malonsäureester hergestellt werden könnten (Chim. geterocikl. Soed. *4*, 614 (1968)), oder — in begrenzten Fällen — durch eine stofflich und technisch aufwendige Variante der Cannizzaro-Reaktion (JA-OS *74* 025248).

Es wurde nun eine allgemein anwendbares Verfahren zur Herstellung dieser substituierten Etheraldole gefunden, bei dem man ein 2-Alkylacrolein mit einem Alkohol (Alkanol) und — vorzugsweise wäßrigem — Formaldehyd in Gegenwart eines tertiären Amins, vorzugsweise eines Alkylamins, das einen $pK_B$-Wert oberhalb von 6 (z. B. 9 bis 11) besitzt, umsetzt.

Bezogen auf 2-Alkylacrolein wird Formaldehyd z. B. im Mol-Verhältnis 1 bis 1,5 : 1 angewandt, der Alkohol z. B. im Molverhältnis 4 bis 6 : 1.

Die Reaktion verläuft im allgemeinen im Temperaturbereich von 60 bis 100 °C bei atmosphärischem Druck mit ausreichender Geschwindigkeit und Selektivität ; je nach Verbindung kommt ein Temperaturbereich von Raumtemperatur bis 150 °C in Betracht.

Die nach der Erfindung erhältlichen Verbindungen sind — abgesehen von der zufällig bekannten Verbindung 2-Propoximethyl-2-methyl-hydroxipropanal — neue Stoffe, die einen vorteilhaften Weg über entsprechend substituierte Malonsäure(ester) zu Pflanzenschutzmitteln eröffnen.

Die benötigten 2-Alkylacroleine sind z. B. nach der DE-OS 31 06 557 durch Mannich-Kondensation von n-Alkanalen mit Formaldehyd zugänglich.

Beispiel 1

Herstellung von 2-Methoximethyl-2-hydroximethyl-propanal

Zu einer Mischung von 590 g Methanol, 330 g 30 %iger Formaldehydlösung und 210 g Methacrolein werden innerhalb 10 Minuten 22 g Triethylamin gegeben und die Lösung 8 Stunden bei Siedetemperatur gehalten. Nach Abtrennung der niedersiedenden Komponenten isoliert man durch Vakuumdestillation 312 g der Titelverbindung (≙ 78 % Ausbeute bezogen auf Methacrolein) mit einem Siedepunkt bei 24 mbar von 120 bis 125 °C.

### Beispiel 2

Herstellung von 2-Methoximethyl-2-hydroximethyl-hexanal

Man gibt zu einer Mischung von 550 g Methanol, 500 g 30 %iger Formaldehydlösung und 450 g Butylacrolein während 15 Minuten 37 g Triethylamin und hält dann 15 Stunden bei Rückflußtemperatur.

Durch Vakuumdestillation erhält man 206 g 2-Methoximethyl-2-hydroximethyl-hexanal (35,4 % bezogen auf umgesetztes Butylacrolein) mit einem Siedepunkt (3 mbar) von 105 bis 107 °C.

### Beispiel 3

Herstellung von 2-Methoximethyl-2-hydroximethyl-butanal

Gemäß Beispiel 1 werden 1 280 g Methanol, 500 g 30 %ige Formaldehydlösung, 328 g Ethylacrolein und 41 g Triethylamin umgesetzt. Durch Vakuumdestillation erhält man 390 g der Titelverbindung (≙ 67 % Ausbeute bezogen auf Ethylacrolein) mit einem Siedepunkt bei 3 mbar von 95 bis 100 °C.

### Anwendungsbeispiel 1

Herstellung von Methoximethyl-methyl-malonsäure

Zu 700 g 65 % Salpetersäure ($d^{20}$ = 1,40) werden 0,1 g $MnCl_2 \cdot H_2O$ gegeben und 300 g 2-Methoximethyl-2-hydroximethyl-propanal bei 35 bis 40 °C innerhalb 90 Minuten zugegeben. Nach beendeter Reaktion werden 150 g Wasser zugesetzt und die gerührte Mischung auf 0 °C abgekühlt. Die dabei auskristallisierte Säure wird abgesaugt und getrocknet.

Ausbeute : 150 g (84 % bezogen auf Aldol)

Schmelzpunkt : etwa 60 °C unter Decarboxilierung

### Anwendungsbeispiel 2

Herstellung von Methoximethyl-propyl-malonsäure

Zu einer Mischung von 356 Gew.-Teilen $HNO_3$ conc. ($d^{20}$ = 1,40) und 0,13 g $MnCl_2 \cdot H_2O$ werden 100 g 2-Methoximethyl-2-hydroximethyl-pentanal bei 35 °C innerhalb 60 Minuten zugegeben.

Aus der auf 5 °C abgekühlten Lösung fallen 57 g Säure in kristalliner Form aus.

Ausbeute : 47 % bezogen auf Aldol

Schmelzpunkt : 90 bis 94 °C unter Decarboxilierung

### Anwendungsbeispiel 3

Herstellung von Methoximethyl-butyl-malonsäure

Gemäß Beispiel 2 werden 304 g $HNO_3$ ($d^{20}$ = 1,40), 0,1 g $MnCl_2 \cdot H_2O$ und 100 g 2-Methoximethyl-2-hydroximethyl-hexanal umgesetzt.

Ausbeute : 83 g (70 % bezogen auf Aldol)

Schmelzpunkt über 100 °C

— decarboxiliert ab 100 °C.

## Patentansprüche

1. Verfahren zur Herstellung von Aldolen der allgemeinen Formel (I)

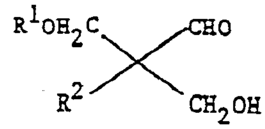

(I)

in der $R^1$ einen verzweigten oder unverzweigten Alkylrest mit bis zu 5 C-Atomen darstellt und $R^2$ einen

verzweigten oder unverzweigten Alkylrest mit bis zu 6 C-Atomen bedeutet, dadurch gekennzeichnet, daß man ein entsprechendes 2-Alkylacrolein $H_2C = CR^2$—CHO, einen entsprechenden Alkohol $R^1OH$ und Formaldehyd in Gegenwart eines tertiären Amins umsetzt.

2. Verfahren nach Anspruch 1, dadurch gekennzeichnet, daß man als tertiäres Amin ein Alkylamin mit einem $pK_B$-Wert von 9 bis 11 verwendet.

3. Verfahren nach Anspruch 1 oder 2, dadurch gekennzeichnet, daß man Formaldehyd in Form seiner wäßrigen Lösung verwendet.

4. Aldol der allgemeinen Formel I

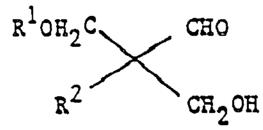

(I)

ausgenommen die Verbindung 2-Propoximethyl-2-methyl-3-hydroxipropan-1-al.

**Claims**

1. A process for the preparation of an aldol of the general formula (I)

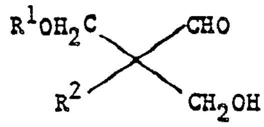

(I)

where $R^1$ is a straight-chain or branched alkyl radical of up to 5 carbon atoms, and $R^2$ is a straight-chain or branched alkyl radical of up to 6 carbon atoms, wherein an appropriate 2-alkylacrolein ($H_2C = CR^2$—CHO), an appropriate alcohol ($R^1OH$) and formaldehyde are reacted in the presence of a tertiary amine.

2. A process as claimed in claim 1, wherein the tertiary amine used is an alkylamine having a $pK_B$ of from 9 to 11.

3. A process as claimed in claim 1 or 2, wherein formaldehyde is used in the form of its aqueous solution.

4. An aldol of the general formula I

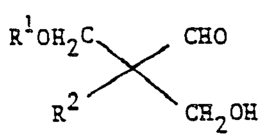

(I)

excluding the compound 2-propoxymethyl-2-methyl-3-hydroxypropan-1-al.

**Revendications**

1. Procédé de préparation d'aldols de formule générale (I)

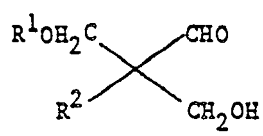

(I)

dans laquelle $R^1$ représente un reste alkyle, ramifié ou non ramifié, ayant jusqu'à 5 atomes C et $R^2$ un reste alkyle ramifié ou non ramifié ayant jusqu'à 6 atomes C, caractérisé par le fait que l'on fait réagir une 2-alkylacroléine $H_2C = CR^2$—CHO correspondante, un alcool $R^1OH$ correspondant et une formaldéhyde, en présence d'une amine tertiaire.

2. Procédé selon la revendication 1, caractérisé par le fait que l'on utilise, comme amine tertiaire, une alkylamine d'un $pK_B$ de 9 à 11.

3. Procédé selon la revendication 1 ou 2, caractérisé par le fait que l'on utilise de la formaldéhyde sous forme d'une solution aqueuse.

4. Aldol de formule générale I

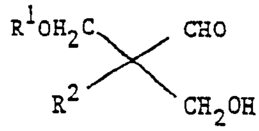

à l'exception du composé 2-propoxyméthyl-2-méthyl-3-hydroxypropan-1-al